# FASCICULE DE BREVET EUROPEEN

(11) **EP 1 012 618 B1**
(45) Date de publication et mention de la délivrance du brevet: **01.02.2006**
(21) Numéro de dépôt: 99929442.4
(22) Date de dépôt: 08.07.1999
(51) Int. Cl.: G01R 33/44, G01N 15/08

(54) **CARACTERISATION D'UN MILIEU PERMEABLE POREUX PAR RMN DE GAZ POLARISE**
CHARAKTERISIERUNG VON EINEM PERMEABLEN PORÖSEN MEDIUM MITTELS GASPOLARIZIERTER KERNSPINRESONANZ
METHOD FOR CHARACTERISING A POROUS PERMEABLE MEDIUM BY POLARISED GAS NMR

(30) Priorité: 10.07.1998 FR 9808921
(43) Date de publication de la demande: 28.06.2000
(73) Titulaire: COMMISSARIAT A L'ENERGIE ATOMIQUE, 75015 Paris (FR)
(72) Inventeur: LOCATELLI, Marcel, F-38330 Montbonnot (FR)
(74) Mandataire: Audier, Philippe André
(86) Numéro de dépôt international: PCT/FR1999/001662
(87) Numéro de publication internationale: WO 2000/003261

(56) Documents cités:
- US-A- 3 850 040
- GREGORY D M ET AL: "Pore-structure determinations of silica aerogels by /sup 129/Xe NMR spectroscopy and imaging" JOURNAL OF MAGNETIC RESONANCE, APRIL 1998, ACADEMIC PRESS, USA, vol. 131, no. 2, pages 327-335, XP002097738 ISSN 1090-7807
- MANSFELD M ET AL: "The use of /sup 129/Xe NMR exchange spectroscopy for probing the microstructure of porous materials" CHEMICAL PHYSICS LETTERS, 1 OCT. 1993, NETHERLANDS, vol. 213, no. 1-2, pages 153-157, XP002097739 ISSN 0009-2614
- PASQUIER V ET AL: "/sup 129/Xe NMR as a probe of the dynamics of gas confined in porous Vycor" THIRD INTERNATIONAL MEETING ON RECENT ADVANCES IN MR APPLICATIONS TO POROUS MEDIA, LOUVAIN LA NEUVE, BELGIUM, 3-6 SEPT. 1995, vol. 14, no. 7-8, pages 971-973, XP002097740 ISSN 0730-725X, Magnetic Resonance Imaging, 1996, Elsevier, USA

## Description

### Domaine technique

La présente invention concerne la caractérisation de milieux perméables poreux, par exemple des objets en bois attaqués par des parasites.

### Etat de la technique antérieure

Il est connu, par exemple par le document US-A-5 357 063 d'utiliser l'énergie acoustique afin de détecter et d'identifier des objets enterrés dans le sol. De l'énergie acoustique est injectée dans le sol et on recueille un signal réfléchi qui est traité pour délivrer une image représentative de l'énergie réfléchie. Cette technique, qui s'applique plus particulièrement à la détection d'objets enterrés, est valable pour les milieux peu poreux. Si le milieu examiné est trop poreux, il se pose des problèmes de propagation de l'énergie acoustique qui influent sur la précision de la méthode.

Le brevet US 5 285 688 implique aussi une méthode acoustique. Il divulgue un système pour la détection d'insectes destructeurs du bois, en particulier un système qui détecte les émissions acoustiques produites par ces insectes et qui sont révélatrices de leur présence. Ce système est basé sur l'écoute des insectes en activité et nécessite par conséquent la présence d'insectes vivants, ce qui n'est pas toujours le cas du milieu que l'on veut caractériser.

On peut encore caractériser un milieu perméable poreux par des méthodes impliquant l'imprégnation du milieu par un liquide dense, tel que le mercure, ou par de l'eau. Ces méthodes nécessitent la présence obligatoire d'un liquide. Elles s'appliquent donc plus particulièrement à des domaines où un liquide est déjà présent dans le milieu concerné, par exemple dans le domaine du forage (cf. les brevets US 5 309 098 et US 5 610 522). Dans d'autres domaines d'application, comme celui relatif aux oeuvres d'art, il y a un risque de détérioration du milieu à caractériser.

Pour éviter la présence d'un liquide, l'imagerie RMN, à partir de la résonance de l'hélium 3, a été utilisée pour visualiser par exemple la porosité des poumons. Cette technique a été divulguée dans les articles suivants :
- "Voir les poumons grâce à l'hélium" de M. LEDUC et E. OTTEN, paru dans la revue La Recherche, n° 287, mai 1996, pages 41-43 ;
- "Low-field ³He nuclear magnetic resonance in human lungs" de L. DARRASSE, G. GUILLOT, P.-J. NACHER et G. TASTEVIN, C.R. Acad. Sci. Paris, t. 324, série IIb, p. 691-700, 1997. Elle consiste à faire inhaler à un patient de l'hélium 3 polarisé et à visualiser le gaz inhalé par résonance magnétique nucléaire. Dans cette technique, il est nécessaire de réaliser une imagerie haute résolution pour mesurer la porosité à partir de la mesure des dimensions des pores sur les images, ce qui nécessite des moyens expérimentaux lourds, complexes et onéreux.

Il est aussi connu de caractériser un milieu poreux, par la résonance magnétique nucléaire, d'un gaz polarisé introduit dans le milieu (voir Journal of Magnetic Resonance, vol 131, pages 327-335, 1998).

### Exposé de l'invention

Pour remédier aux inconvénients de l'art antérieur, il est proposé une méthode de caractérisation qui utilise la RMN de gaz polarisés, tels que ³He ou ¹²⁹Xe.

L'invention a donc pour objet une méthode de caractérisation d'un objet perméable poreux, comprenant les étapes suivantes :
- mise en place de l'objet dans une enveloppe étanche,
- mise en dépression de l'enveloppe contenant ledit objet,
- mesure de la pression résiduelle régnant dans l'enveloppe mise en dépression,
- introduction d'une quantité de gaz polarisé dans l'enveloppe mise en dépression,
- mesure de la pression régnant dans l'enveloppe suite à l'introduction de ladite quantité de gaz polarisé,
- détermination de la densité du gaz polarisé introduit dans l'enveloppe à partir desdites mesures de pression,
- mesure par RMN de la quantité massique de gaz polarisé contenu dans un volume connu de l'objet placé dans l'enveloppe étanche,
- détermination de la porosité de l'objet par calcul du rapport quantité massique de gaz polarisé/densité du gaz polarisé introduit dans l'enveloppe.

La mise en place de l'objet peut s'effectuer dans une enveloppe constituée par une enceinte rigide. Elle peut s'effectuer dans une enveloppe suffisamment souple pour que sa mise en dépression provoque l'application de l'enveloppe sur l'objet. La quantité de gaz polarisé introduite dans l'enveloppe peut alors être telle que l'enveloppe reste appliquée sur l'objet.

Le gaz polarisé peut être choisi parmi ³He et ¹²⁹Xe.

La mesure RMN peut être effectuée par un système RMN de surface. Cette mesure peut être effectuée par un système RMN de surface mettant en jeu des séquences CPMG, ce qui permet de déterminer les dimensions moyennes des porosités.

La mesure RMN peut aussi être effectuée par un système RMN de volume.

L'invention a aussi pour objet un dispositif de caractérisation d'un objet perméable poreux, comprenant :
- une enveloppe étanche apte à recevoir ledit objet,
- des moyens de mesure de la pression régnant dans l'enveloppe étanche,
- des moyens permettant la mise en dépression de ladite enveloppe étanche,
- des moyens permettant d'introduire une quantité de gaz polarisé dans l'enveloppe étanche mise en dépression,
- des moyens de mesure par RMN de la quantité massique de gaz polarisé contenu dans un volume connu de l'objet placé dans l'enveloppe étanche,
- des moyens de détermination de la densité du gaz polarisé introduit dans l'enveloppe à partir de données fournies par les moyens de mesure, et de détermination de la porosité de l'objet à partir de la mesure de la quantité massique de gaz polarisé et de la densité du gaz polarisé introduit dans l'enveloppe.

### Brève description des dessins

L'invention sera mieux comprise et d'autres avantages et particularités apparaîtront à la lecture de la description qui va suivre, donnée à titre d'exemple non limitatif, accompagnée des figures annexées parmi lesquelles :
- la figure 1 est illustrative d'un dispositif de caractérisation d'un objet perméable poreux selon la présente invention et comprenant une enveloppe étanche constituée d'une enceinte rigide,
- la figure 2 est illustrative d'un dispositif de caractérisation d'un objet perméable poreux selon la présente invention, comprenant une enveloppe étanche souple et un système RMN de surface,
- la figure 3 est illustrative d'un dispositif de caractérisation d'un objet perméable poreux selon la présente invention, comprenant une enveloppe étanche souple et un système RMN de volume.

### Description détaillée de modes de réalisation de l'invention

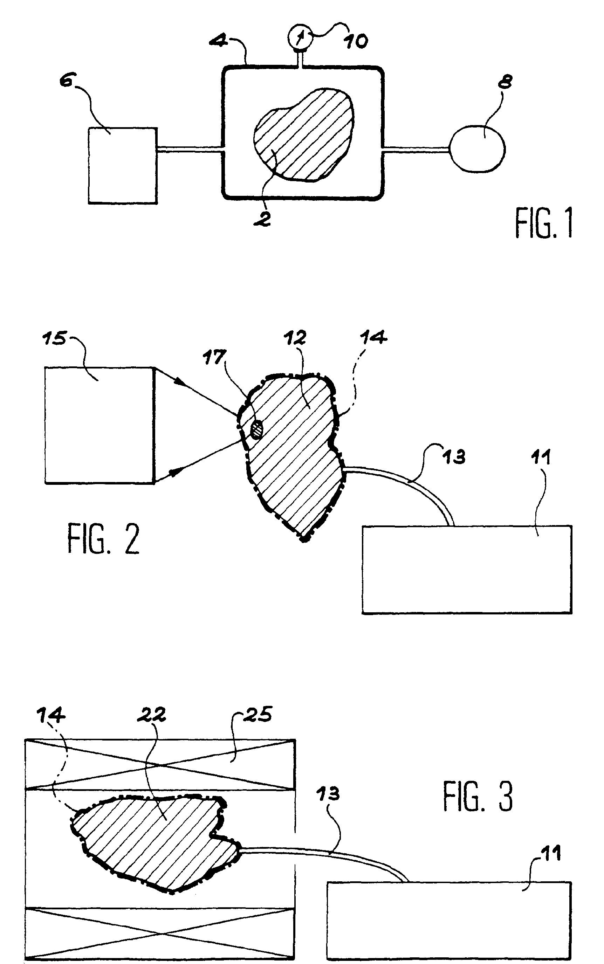

Selon l'invention, le gaz polarisé est introduit dans l'objet à caractériser à une pression contrôlée. Pour cela, et comme le montre la figure 1, l'objet 2 est placé dans une enveloppe étanche 4 reliée d'une part à un système de pompage 6 et d'autre part à un réservoir de gaz polarisé 8. Un manomètre 10 permet de mesurer la pression régnant à l'intérieur de l'enveloppe étanche 4.

Dans le mode de réalisation représenté à la figure 1, l'enveloppe étanche 4 est constituée par une enceinte rigide.

Le gaz polarisé utilisé est par exemple ³He que l'on polarise par une méthode connue (voir par exemple l'article cité plus haut de M. LEDUC et E. OTTEN).

La mesure RMN s'effectue dans un volume connu de l'objet à partir, par exemple, d'un système de surface tel que celui décrit dans le brevet US 5 610 522. Cette mesure permet de connaître la quantité massique de gaz contenue dans ce volume de l'objet.

Après avoir placé l'objet 2 dans l'enveloppe étanche 4, celle-ci est mise en dépression. Une fois l'enveloppe mise en dépression, on mesure la pression résiduelle grâce au manomètre 10. Du gaz polarisé, provenant du réservoir 8, est alors introduit dans l'enveloppe étanche 4. On mesure alors la pression régnant à l'intérieur de l'enveloppe 4. De la différence entre la pression après introduction du gaz polarisé et la pression résiduelle, on déduit la densité du gaz polarisé présent à l'intérieur de l'enveloppe étanche.

A partir de la quantité massique obtenue par la mesure RMN et de la densité du gaz polarisé, on déduit le volume de gaz polarisé dans l'objet, donc la porosité moyenne dans le volume de mesure.

L'enveloppe étanche peut être rigide ou souple. L'utilisation d'une enveloppe souple permet de limiter le volume de gaz polarisé à introduire pour obtenir une pression de gaz donnée. Le signal RMN recueilli étant proportionnel à la densité de gaz, il est avantageux de la maximiser. En outre, cette solution permet de limiter l'espace entre l'objet et le capteur RMN au minimum en utilisant une pression finale inférieure à la pression atmosphérique ambiante, ce qui permet d'appliquer l'enveloppe souple sur l'objet. Cette configuration est importante pour les systèmes de surface tels que celui décrit dans le brevet US 5 610 522 pour lesquels la distance entre le système RMN et le volume de mesure est assez réduite (quelques centimètres par exemple).

La figure 2 est illustrative d'un dispositif selon l'invention comprenant une enveloppe souple 14 plaquée sur l'objet 12 à caractériser lorsque l'intérieur de l'enveloppe est mis en dépression.

Sur cette figure, on a également représenté sous la référence 11 un groupe de pompage avec réserve de gaz et manomètre, relié à l'intérieur de l'enveloppe souple 14 par une liaison pneumatique 13. La référence 15 désigne un système RMN de surface en cours de mesure sur un volume donné 17 de l'objet 12.

Comme pour une technique développée dans le domaine pétrolier (cf. le brevet US 5 309 098), l'utilisation d'un système RMN de surface mettant en jeu des séquences CPMG (Carr-Purcell-Meiboom-Gill) permet, à partir de l'analyse de la décroissance des signaux, de déterminer les dimensions moyennes des porosités.

L'utilisation d'un système RMN de volume permet de balayer l'ensemble de l'objet à caractériser. Ce cas est illustré par la figure 3. L'objet 22 à caractériser est placé dans l'enveloppe souple 14 reliée au groupe 11 par la liaison pneumatique 13 comme pour la figure 2. La totalité de l'objet 22, enfermé dans l'enveloppe souple 14, est placée à l'intérieur du système RMN de volume 25. Ce dispositif est plutôt réservé à des objets de petites dimensions.

## Revendications

1. Méthode de caractérisation d'un objet perméable poreux (2, 12, 22), comprenant les étapes suivantes :
- mise en place de l'objet dans une enveloppe étanche (4, 14),
- mise en dépression de l'enveloppe contenant ledit objet,
- mesure de la pression résiduelle régnant dans l'enveloppe mise en dépression,
- introduction d'une quantité de gaz polarisé dans l'enveloppe mise en dépression,
- mesure de la pression régnant dans l'enveloppe suite à l'introduction de ladite quantité de gaz polarisé,
- détermination de la densité du gaz polarisé introduit dans l'enveloppe à partir desdites mesures de pression,
- mesure par RMN de la quantité massique de gaz polarisé contenu dans un volume connu de l'objet placé dans l'enveloppe étanche,
- détermination de la porosité de l'objet par calcul du rapport quantité massique de gaz polarisé/densité du gaz polarisé introduit dans l'enveloppe.

2. Méthode selon la revendication 1, **caractérisée en ce que** la mise en place de l'objet (2) s'effectue dans une enveloppe (4) constituée par une enceinte rigide.

3. Méthode selon la revendication 1, **caractérisée en ce que** la mise en place de l'objet (12, 22) s'effectue dans une enveloppe (14) suffisamment souple pour que sa mise en dépression provoque l'application de l'enveloppe sur l'objet.

4. Méthode selon la revendication 3, **caractérisée en ce que** la quantité de gaz polarisé introduite dans l'enveloppe est telle que l'enveloppe (14) reste appliquée sur l'objet (12, 22).

5. Méthode selon l'une quelconque des revendications 1 à 4, **caractérisée en ce que** l'étape d'introduction d'une quantité de gaz polarisé consiste à introduire un gaz choisi parmi ³He et ¹²⁹Xe.

6. Méthode selon l'une quelconque des revendications 1 à 5, **caractérisée en ce que** la mesure par RMN est effectuée par un système RMN de surface (15) .

7. Méthode selon la revendication 6, **caractérisée en ce que** la mesure par RMN est effectuée par un système mettant en jeu des séquences CPMG, ce qui permet de déterminer les dimensions moyennes des porosités.

8. Méthode selon l'une quelconque des revendications 1 à 5, **caractérisée en ce que** la mesure par RMN est effectuée par un système RMN de volume (25).

9. Dispositif de caractérisation d'un objet perméable poreux (2, 12, 22), comprenant :
- une enveloppe étanche (4, 14) apte à recevoir ledit objet,
- des moyens (10) de mesure de la pression régnant dans l'enveloppe étanche,
- des moyens (6) permettant la mise en dépression de ladite enveloppe étanche,
- des moyens (8) permettant d'introduire une quantité de gaz polarisé dans l'enveloppe étanche mise en dépression,
- des moyens de mesure par RMN de la quantité massique de gaz polarisé contenu dans un volume connu de l'objet placé dans l'enveloppe étanche,
- des moyens de détermination de la densité du gaz polarisé introduit dans l'enveloppe à partir de données fournies par les moyens de mesure, et de détermination de la porosité de l'objet à partir de la mesure de la quantité massique de gaz polarisé et de la densité du gaz polarisé introduit dans l'enveloppe.

10. Dispositif selon la revendication 9, **caractérisé en ce que** ladite enveloppe étanche (4) est une enceinte rigide.

11. Dispositif selon la revendication 9, **caractérisé en ce que** ladite enveloppe étanche (14) est une enveloppe suffisamment souple pour que sa mise en dépression provoque l'application de l'enveloppe sur l'objet (2, 12).

12. Dispositif selon la revendication 11, **caractérisé en ce que** ladite enveloppe étanche (14) est une enveloppe suffisamment souple pour rester appliquée sur l'objet (2, 12) après l'introduction de ladite quantité de gaz polarisé.

13. Dispositif selon l'une quelconque des revendications 9 à 12, **caractérisé en ce que** lesdits moyens (8) d'introduction d'une quantité de gaz polarisé sont des moyens d'introduction d'un gaz choisi parmi ³He et ¹²⁹Xe.

14. Dispositif selon l'une quelconque des revendications 9 à 13, **caractérisé en ce que** lesdits moyens de mesure par RMN comprennent un système RMN de surface (15).

15. Dispositif selon la revendication 14, **caractérisé en ce que** ledit système RMN de surface (15) est un système susceptible de mettre en jeu des séquences CPMG afin de déterminer les dimensions moyennes des porosités.

16. Dispositif selon l'une quelconque des revendications 9 à 13, **caractérisé en ce que** lesdits moyens de mesure par RMN comprennent un système RMN de volume (25) .

## Claims

1. Method for characterising a porous permeable object (2, 12, 22) including the following steps:
- placing the object in an air-tight container (4, 14),
- creating a partial vacuum in the container containing the aforesaid object,
- measuring the residual pressure in the container
in which the partial vacuum is formed,
- introducing a quantity of polarised gas in the container in partial vacuum,
- measuring the pressure in the container after introduction of the aforesaid quantity of polarised gas,
- determination of the density of the polarised gas introduced into the container from the aforesaid pressure measurements,
- NMR measurement of the mass quantity of polarised gas contained in a known volume of the object placed in the air-tight container,
- determination of the porosity of the object by calculation of ratio of the mass quantity of polarised gas to the density of the polarised gas introduced into the container.

2. Method according to claim 1, **characterised in that** the object (2) is placed in a rigid container (4).

3. Method according to claim 1, **characterised in that** the object (12, 22) is placed in a container (14) which is sufficiently flexible so that the depressurising causes the container to adhere to the object.

4. Method according to claim 3, **characterised in that** the quantity of polarised gas introduced into the container is such that the container (14) continues to adhere to the object (12, 22).

5. Method according to any of claims 1 to 4, **characterised in that** the step of introduction of a quantity of polarised gas involves introduction of a gas chosen from ³He or ¹²⁹Xe.

6. Method according to any of claims 1 to 5, **characterised in that** the NMR measurement is done by a NMR area system (15).

7. Method according to claim 6, **characterised in that** the NMR measurement is done by a system using CPMG sequences, thus allowing for determination of average porosity dimensions.

8. Method according to any of claims 1 to 5, **characterised in that** the NMR measurement is done by a NMR volume system (25).

9. Characterisation device for a porous permeable object (2, 12, 22) including;
- an air-tight container (4, 14) apt to receive the aforesaid object,
- means (10) for measurement of the pressure in the air-tight container,
- means (6) allowing for creation of a partial vacuum in the aforesaid air-tight container,
- means (8) for introducing a quantity of polarised gas into the depressurised air-tight container,
- means for NMR measurement of the mass quantity of polarised gas contained in a known volume of the object placed in the air-tight container,
- means for determination of the density of the polarised gas introduced into the container from data supplied by the means of measurement, and determination of the porosity of the object from measurement of the mass quantity of the polarised gas and the density of the polarised gas introduced into the container.

10. Device according to claim 9, **characterised in that** the aforesaid air-tight container (4) is rigid.

11. Device according to claim 9, **characterised in that** the aforesaid air-tight container (14) is sufficiently flexible so that the depressurising causes the container to adhere to the object (2, 12).

12. Device according to claim 11, **characterised in that** the aforesaid air-tight container (14) is sufficiently flexible so that it continues to adhere to the object (2, 12) after introduction of the aforesaid quantity of polarised gas.

13. Device according to any of claims 9 to 12, **characterised in that** the aforesaid means (8) for introducing a quantity of polarised gas are means for introducing a gas chosen from ³He or ¹²⁹Xe.

14. Device according to any of claims 9 to 13, **characterised in that** the aforesaid means of NMR measurement include an NMR area system (15).

15. Device according to claim 14, **characterised in that** the aforesaid NMR area system (15) is a system which can use CPMG sequences in order to determine the average porosity dimensions.

16. Device according to any of claims 9 to 13, **characterised in that** the aforesaid means of NMR measurement include an NMR volume system (25).

## Patentansprüche

1. Verfahren zur Charakterisierung eines porösen permeablen Objekts (2, 12, - 22), die folgenden Schritte umfassend:
- Einschließen des Objekts in eine dichte Hülle (4, 14),
- Erzeugen eines Unterdrucks in der das Objekt enthaltenden Hülle,
- Messen des in der Hülle nach dem Erzeugen des Unterdrucks herrschenden Restdrucks,
- Einspeisen einer Menge eines polarisierten Gases in die Unterdruck aufweisende Hülle,
- Messen des in der Hülle infolge der Einspeisung der genannten Menge polarisierten Gases herrschenden Drucks,
- Bestimmen der Dichte des in die Hülle eingespeisten polarisierten Gases aufgrund der genannten Druckmessungen,
- Messen der Massenmenge des in einem bekannten Volumen des in der dichten Hülle eingeschlossenen Objekts enthaltenen polarisierten Gases mittels NMR,
- Bestimmen der Porosität des Objekts durch Berechnung des Verhältnisses der Massenmenge des polarisierten Gases zur Dichte des in die Hülle eingespeisten polarisierten Gases.

2. Verfahren nach Anspruch 1, **dadurch gekennzeichnet, dass** das Objekt (2) in eine Hülle (4) eingeschlossen wird, gebildet durch einen steifen Behälter.

3. Verfahren nach Anspruch 1, **dadurch gekennzeichnet, dass** das Objekt (12, 22) in eine Hülle (14) eingeschlossen wird, die so nachgiebig ist, dass sie sich an das Objekt schmiegt, wenn in ihr ein Unterdruck erzeugt wird.

4. Verfahren nach Anspruch 3, **dadurch gekennzeichnet, dass** die in die Hülle eingespeiste Menge polarisierten Gases so ist, dass die Hülle (14) an das Objekt (12, 22) geschmiegt bleibt.

5. Verfahren nach einem der Ansprüche 1 bis 4, **dadurch gekennzeichnet, dass** der Schritt zur Einspeisung einer Menge polarisierten Gases darin besteht, ein zwischen ³He und ¹²⁹Xe ausgewähltes Gas einzuspeisen.

6. Verfahren nach einem der Ansprüche 1 bis 5, **dadurch gekennzeichnet, dass** das Messen mittels NMR durch ein NMR-Oberflächensystem (15) erfolgt.

7. Verfahren nach Anspruch 6, **dadurch gekennzeichnet, dass** das Messen mittels NMR durch ein System erfolgt, das CPMG-Sequenzen benutzt, was ermöglicht, die mittleren Dimensionen der Porositäten zu bestimmen.

8. Verfahren nach einem der Ansprüche 1 bis 5, **dadurch gekennzeichnet, dass** das Messen mittels NMR durch ein NMR-Volumensystem (25) erfolgt.

9. Vorrichtung zur Charakterisierung eines porösen permeablen Objekts (2, 12, 22), umfassend:
- eine dichte Hülle (4, 14) zur Aufnahme des genannten Objekts,
- Einrichtungen (10) zum Messen des in der dichten Hülle herrschenden Drucks,
- Einrichtungen (6) zum Erzeugen eines Unterdrucks in der genannten dichten Hülle,
- Einrichtungen (8) zum Einspeisen einer Menge eines polarisierten Gases in die Unterdruck aufweisende dichte Hülle,
- Einrichtungen zum Messen der Massenmenge des in einem bekannten Volumen des in der dichten Hülle eingeschlossenen Objekts enthaltenen polarisierten Gases mittels NMR,
- Einrichtungen zum Bestimmen der Dichte des in die Hülle eingespeisten polarisierten Gases aufgrund der durch die Messeinrichtungen gelieferten Daten, und zum Bestimmen der Porosität des Objekts aufgrund der Messung der Massenmenge des polarisierten Gases und der Dichte des in die Hülle eingespeisten polarisierten Gases.

10. Vorrichtung nach Anspruch 9, **dadurch gekennzeichnet, dass** die genannte dichte Hülle (4) ein steifer Behälter ist.

11. Vorrichtung nach Anspruch 9, **dadurch gekennzeichnet, dass** die genannte dichte Hülle (14) eine so nachgiebig Hülle ist, dass sie sich an das Objekt (2, 12) schmiegt, wenn in ihr ein Unterdruck erzeugt wird.

12. Vorrichtung nach Anspruch 11, **dadurch gekennzeichnet, dass** die genannte dichte Hülle (14) eine so nachgiebig Hülle ist, dass sie nach der Einspeisung der genannten Menge polarisierten Gases an das Objekt (2, 12) geschmiegt bleibt.

13. Vorrichtung nach einem der Ansprüche 9 bis 12, **dadurch gekennzeichnet, dass** die Einrichtungen (8) zur Einspeisung einer Menge polarisierten Gases Einrichtungen zur Einspeisung eines zwischen ³He und ¹²⁹Xe ausgewählten Gases sind.

14. Vorrichtung nach einem der Ansprüche 9 bis 13, **dadurch gekennzeichnet, dass** die genannten Einrichtungen zum Messen mittels NMR ein NMR-Oberflächensystem (15) umfassen.

15. Vorrichtung nach Anspruch 14, **dadurch gekennzeichnet, dass** das genannte NMR-Oberflächensystem (15) ein System ist, das CPMG-Sequenzen benutzen kann, um die mittleren Dimensionen der Porositäten zu bestimmen.

16. Vorrichtung nach einem der Ansprüche 9 bis 13, **dadurch gekennzeichnet, dass** die genannten NMR-Messeinrichtungen ein NMR-Volumensystem (25) umfassen.
